# EUROPEAN PATENT APPLICATION

(11) **EP 4 300 942 A1**
(43) Date of publication of application: **03.01.2024**
(21) Application number: 22762615.7
(22) Date of filing: 04.03.2022
(51) Int. Cl.: H04N 5/235, A61B 8/12

(54) **AUTOMATIC GAIN COMPENSATION METHOD AND SYSTEM FOR IMAGING, AND STORAGE MEDIUM AND ULTRASONIC ENDOSCOPE**

(30) Priority: 05.03.2021 CN 202110246262
(71) Applicant: Innermedical Co., Ltd, Shenzhen, Guangdong 518055 (CN)
(72) Inventor: BAI, Xiaosong, Shenzhen, Guangdong 518055 (CN); TU, Shipeng, Shenzhen, Guangdong 518055 (CN)
(74) Representative: J A Kemp LLP
(86) International application number: PCT/CN2022/079160
(87) International publication number: WO 2022/184153

(57) **Abstract**

This application provides an imaging automatic gain compensation method, system and storage medium, and ultrasound endoscope, which comprises the following steps: applying a current TGC curve to obtain a current frame image; and setting an attenuation coefficient of a probe acoustic wave in water and an attenuation coefficient of a probe acoustic wave in human tissue; locating a depth region containing a tissue image according to the current frame image, and obtaining a depth range of a tissue part segment in a far field segment and a depth range of a water coupling segment in a near field segment by means of signal and image processing, and making the corresponding gain compensation respectively; according to a different attenuation of the depth range of the water coupling segment and the tissue part segment, obtaining a corresponding full-depth attenuation curve, and updating a corresponding TGC curve; applying the TGC curve of a previous frame in next frame processing and repeating the above steps. By adopting the technical scheme of this application, the local gain is automatically adjusted in real time, and the gain is compensated by different strategies for near field and far field, and a soft brightness image can be obtained.

## Description

### Cross reference to related application

The present application claims priority to Chinese Patent Application No. 202110246262.6 filed to CNIPA on March 05, 2021 and entitled " Imaging Automatic Gain Compensation Method, System and Storage medium, and Ultrasonic Endoscope ", the entire contents of which are incorporated herein by reference.

### Technical field

This application relates to a technical field of ultrasonic endoscopic imaging, in particular to an imaging automatic gain compensation method , system and storage medium, and ultrasonic endoscope.

### Technical background

EUS (Endoscopic Ultrasonography) is a digestive tract examination technology combining endoscope and ultrasound. A miniature high-frequency ultrasonic probe is placed at the top of an endoscope. After the endoscope is inserted into the body cavity, while the gastrointestinal mucosal lesions are directly observed by the endoscope, real-time scanning can be performed by using endoscopic ultrasound to obtain ultrasound images for hierarchical structure, histological features and neighboring organs of the gastrointestinal tract, thus further improving the diagnostic level of endoscope and ultrasound.

After more than 20 years of clinical practice, endoscopic ultrasonography is becoming more and more mature, and its application scope is constantly expanding. The appearance of endoscopic ultrasonography has made a leap development of endoscopic technology. The endoscopic ultrasonography can be used for differential diagnosis of the nature of lesions growing under the mucosa of the digestive tract wall, preoperative analysis on digestive tract tumors to determine the depth and scope of invasion thereof, differentiation of benign and malignant ulcers, and diagnosis of pancreatic and biliary system tumors, especially for smaller tumors with high accuracy, and diagnosis of chronic pancreatitis is also better than other imaging examinations. Diagnostic endoscopic ultrasonography has a wide application prospect in esophageal cancer, gastric lymphoma cancer, gastric cancer, submucosal tumors, esophageal and gastric varices, pancreatic and biliary diseases, etc. With the popularization of endoscopic ultrasonography in clinical practice, its application is becoming more and more widely, and it plays an important role especially for the preoperative staging of digestive tract tumors, clarifying the invasion depth of early gastrointestinal cancer, and reasonably grasping the indications of minimally invasive treatment under endoscopy.

Clinically known as a small probe ultrasound endoscope, it is a mechanical ring scan ultrasound endoscope, its working principle is that a single ultrasonic transducer is rotated under the drive of a motor to generate a ring ultrasound, which can perform 360° scanning. The mechanical circular scanning ultrasonic endoscope transducer has a simple structure, and the single array element can be made very small, and the frequency can also be made very high, usually above 15MHz, so the resolution will be very high, and the early subtle lesions of the tissue and the internal structure of the lesion can be detected. When it is used, the micro-ultrasonic scanning probe is sent into a human body through the biopsy channel of an electronic endoscope. At the same time when the endoscope observes the mucosal surface of the internal organs in the body, the ultrasonic scanning probe can obtain the tomography image of the wall of the human internal organs, and discover the early cancerous and micro-tumors in it, which is currently the best method for diagnosing internal organ lesions in the human body. Compared with large ultrasound endoscope, small probe ultrasound endoscope is simple to use and has a shorter clinic training cycle.

Unlike extracorporeal ultrasound endoscope or large ultrasound endoscope, the small probe ultrasound endoscope is used at a higher frequency, generally from 12 MHZ to 20 MHZ. They have a greater attenuation in human soft tissue, with a penetration depth of 1 to 2cm. However, in actual use, the probe acoustic window is not closely attached to the tissue for imaging as in extracorporeal ultrasound endoscope or large ultrasound endoscope, but has a certain distance from the target site. In clinical practice, the imaging observation is performed by infusing water at the target site or by using the water sac as ultrasonic coupling (for example, the hierarchical structure of the gastric wall is observed in a large stomach cavity). Due to the different attenuation of high-frequency ultrasound in water and in human body, the distance between the acoustic window and the target site changes in real time (in extracorporeal ultrasound can keep the probe still, and the distance between the acoustic window and the target is basically unchanged). The small probe ultrasound endoscope has a larger imaging radius (up to 6cm) and a smaller penetration depth. Its TGC (Time Gain Compensation) is difficult to achieve in a fixed way, and the clinical examination time is relatively urgent, so it needs a real-time automatic gain compensation method according to the distance from the acoustic window to the target and image brightness and darkness.

### Summary

In view of the above technical problems, this application discloses an imaging automatic gain compensation method, system and storage medium, and ultrasonic endoscope, which solves the problem of real-time adaptive gain compensation when the spacing between the acoustic window and the target tissue changes in real time.

Therefore, the technical scheme adopted by this application is as follows:
An imaging automatic gain compensation method, which comprises the following steps:
step S1, applying a current TGC curve to obtain a current frame image; and setting an attenuation coefficient of a probe acoustic wave in water and an attenuation coefficient of a probe acoustic wave in human tissue;
step S2, locating a depth region containing a tissue image according to the current frame image, and obtaining a depth range of a tissue part segment in a far field segment and a depth range of a water coupling segment in a near field segment by means of signal and image processing;
for the depth range of the tissue part, making a corresponding gain compensation by using the attenuation coefficient of a probe acoustic wave in human tissue;
for the depth range of the water coupling segment, making a corresponding gain suppression by using the attenuation coefficient of a probe acoustic wave in water;
step S3, according to the depth range of the water coupling segment and the depth range of the tissue part segment, and an attenuation difference between in water and in a human body, obtaining a corresponding full-depth attenuation curve, and updating a corresponding TGC curve;
step S4, applying the TGC curve of a previous frame in next frame processing, and repeating steps S1 to S3.

As a further improvement of this application, in step S1, setting an attenuation coefficient of a current probe acoustic wave in water as α1, setting an attenuation coefficient of a current probe acoustic wave in a current part of a human body to be examined as α2, according to different frequencies of probe and different ultrasonic attenuation of different tissue parts, the system presets different attenuation coefficients α1 and α2.

As a further improvement of this application, wherein α2>α1. By adopting the technical scheme, the gain is suppressed in the near field segment, and the gain is compensated in the far field segment, to obtain a soft brightness image.

As a further improvement of this application, it is specifically defined that the attenuation coefficient in water is α 1 = 0.1 dB/(cm MHz); The attenuation coefficient in human soft tissue is α 2 = 0.7 dB/(cm MHz). The setting of attenuation coefficient can be flexibly operated as an adjustable and preset parameter of the system according to the examination part and doctor's habits.

The above technical scheme sets the compensation gain according to the attenuation coefficient. However, there is usually a water coupling between a probe acoustic window and a tissue part, and the spacing is not fixed. That is, the attenuation in a depth direction is segmented, the former segment is the attenuation α1 in water, and the latter segment is the attenuation α2 in a human body, so to design the TGC curve, it is necessary to know its depth range in water and in tissue. The initial TGC curve is usually designed as an attenuation curve in water or an attenuation curve in tissue. The update on TGC curve in subsequent frames is to make gradient treatment on the initial TGC curve.

As a further improvement of this application, in step S2, due to a larger difference between a gray scale of the image in water and in tissue, a tissue area and a background (low echo area) are distinguished by gray scale or texture during image processing so as to obtain the depth range of the tissue area. Out of this depth range, and before this depth range (near the near-field segment) it can be considered to be a low attenuation coefficient α1 area in water, and in this depth range it is a high attenuation coefficient α2. Corresponding to the depth direction, the appropriate and smooth gain compensation is made.

As a further improvement of this application, the blind area of the probe is also distinguished when the tissue area and the background area are distinguished. The blind area of the probe is distinguished according to a position in the image corresponding to a physical size of the blind area corresponding to a probe model obtained by predetermined probe identification.

As a further improvement of this application, according to a size of the blind area corresponding to the obtained probe model, setting a near field area as a gain attenuation area (the gain coefficient of TGC < 1) for subsequent reduction of a highlighted blind area signal, and setting a far field as a gain compensation area (the gain coefficient of TGC > 1) for amplifying a weak tissue signal.

As a further improvement of this application, in step S2, when obtaining the depth range of the tissue part, using a union of depth range value sets of continuous multi-frame tissue as a depth position area of the tissue.

As a further improvement of this application, designing a stable TGC curve for the continuous multi-frame to ensure a gradual change in brightness of inter-frame images and intra-frame images.

As a further improvement of this application, in order to ensure real-time adjustment, a computing platform such as FPGA or GPU can be used to accelerate image processing and TGC curve update.

As a further improvement of this application, the method to adjust and update the TGC curve can be an analog way for adjusting, that is, using a signal amplifier with variable gain to adjust an amplitude of an analog signal before sampling, and it can be adjusted in a digital way after acquisition, that is, using a gain coefficient of the curve to adjust a numerical value of data.

As a further improvement of this application, in step S3, when the depth range area of the detected tissue part segment is smaller than a preset value, the TGC curve being not updated.

This application also discloses an imaging automatic gain compensation system, which comprises:
a current frame obtaining module, configured to apply a current TGC curve to obtain a current frame image;
an attenuation coefficient setting module, configured to set an attenuation coefficient of a probe acoustic wave in water and an attenuation coefficient of a probe acoustic wave in human tissue;
a signal and image processing module, configured to locate a depth region containing a tissue image according to the current frame image, and obtain a depth range containing a tissue part and a depth range of a water coupling segment;
a gain compensation module, configured to make a corresponding gain compensation by using the attenuation coefficient of a probe acoustic wave in human tissue for the depth range containing the tissue part; and make a corresponding gain suppression by using the attenuation coefficient of a probe acoustic wave in water for the depth range of the water coupling segment;
a TGC curve adjusting and updating module, configured to according to the depth range of the water coupling segment and the depth range of the tissue part segment, and an attenuation difference between in water and in a human body, obtain a corresponding full-depth attenuation curve, and update a corresponding TGC curve.

As a further improvement of this application, the attenuation coefficient setting module, is configured to set the attenuation coefficient of a probe acoustic wave in water as α1, and set the attenuation coefficient of a probe acoustic wave in a human body as α2, wherein α2>α1. As a further improvement of this application, the signal and image processing module is configured to distinguish a tissue area and a background (low echo area) by gray scale or texture during image processing.

As a further improvement of this application, the signal and image processing module is configured to distinguish between tissue area and background while distinguishing the probe blind area. Furthermore, the blind area of the probe is distinguished according to a position in the image corresponding to a physical size of the blind area corresponding to a probe model obtained by predetermined probe identification, when performing image processing; and according to a size of the blind area corresponding to the obtained probe model, a near field area is set as a gain attenuation area (the gain coefficient of TGC < 1) for subsequent reduction of a highlighted blind area signal, and a far field is set as a gain compensation area (the gain coefficient of TGC > 1) for amplifying a weak tissue signal.

As a further improvement of this application, the signal and image processing module, is configured to when obtaining the depth range of the tissue part, use a union of depth range value sets of continuous multi-frame tissue as a depth position area of the tissue and furthermore design a stable TGC curve for a continuous multi-frame to ensure a gradual change in brightness of inter-frame images and intra-frame images.

As a further improvement of this application, when the depth range area of the detected tissue part segment is smaller than a preset value, the TGC curve adjusting and updating module is configured to do not update the TGC curve.

As a further improvement of this application, the TGC curve adjusting and updating module is configured to update the TGC curve by using a signal amplifier with variable gain to adjust an amplitude of an analog signal before sampling, or by using the gain coefficient of the curve to adjust a numerical value of data after acquisition.

As a further improvement of this application, in order to ensure real-time adjustment, the system further comprises a computing platform such as FPGA or GPU, which is configured to accelerate image processing and TGC curve update.

This application also discloses a computer-readable storage medium, wherein instructions are stored in the computer-readable storage medium, and when the instructions are run on an ultrasonic endoscopic imaging device, the ultrasonic endoscopic imaging device is caused to perform the imaging automatic gain compensation method described in any of the preceding items.

This application also discloses an ultrasonic endoscope, comprising a memory, a processor, and a computer program stored in the memory and executable on the processor, when the processor executes the computer program, the imaging automatic gain compensation method described in any of the above items is implemented.

Compared with the prior art, this application has the beneficial effects that:
The technical scheme of this application can automatically adjust the local gain in real time after adjusting the global gain, and adopt different strategies to gain for a near field and a far field. In general, the gain is suppressed in the near field and compensated in the far field, and a soft brightness image can be obtained. The technical scheme of this application can assist the doctor to automatically adjust the brightness of the target in the image when the patient is examined and operated, so as to facilitate the doctor's operation and diagnosis.

### Brief description of the drawings

Fig. 1 is a flowchart of an imaging automatic gain compensation method of this application.
Fig. 2 is a schematic diagram of the attenuation coefficient of ultrasound in tissue and in water according to the embodiment of this application.
Fig. 3 is a schematic diagram of extracting the depth range of a tissue area according to an embodiment of this application.
Fig. 4 is a schematic diagram of extracting the depth range of a tissue area according to another embodiment of this application.
Fig. 5 is a schematic diagram of the water-tissue composite attenuation according to the embodiment of this application.

### Details of description

Hereinafter, some embodiments of this application are described in further detail.

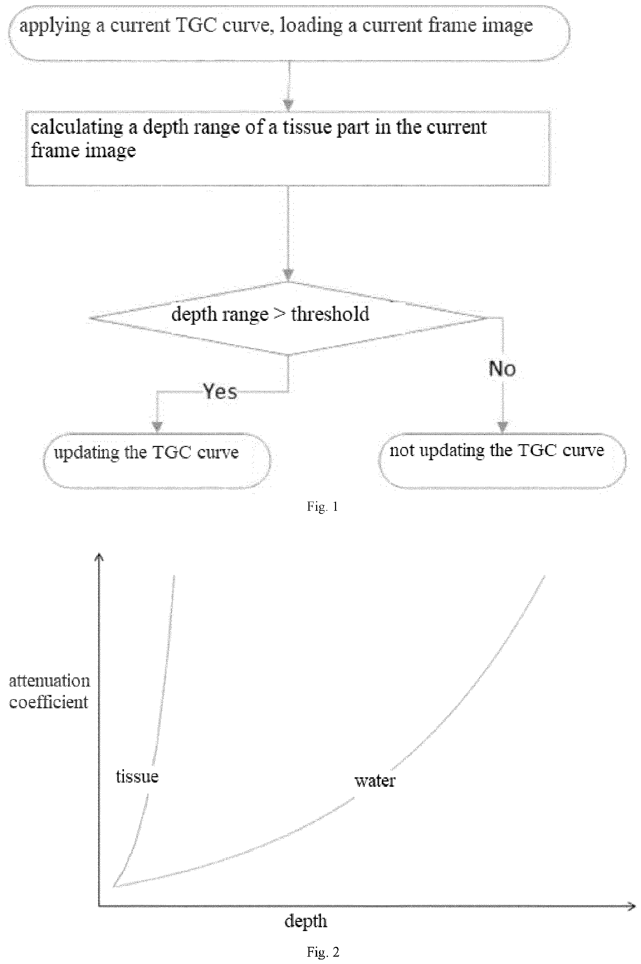

An imaging automatic gain compensation method automatically adjusts the local gain in real time after adjusting the global gain, the gain is suppressed in a near field and compensated in a far field, and a soft brightness image can be obtained. The detailed steps are shown in Fig. 1.

Firstly, the current TGC curve is applied to obtain the current frame image. Defining attenuation coefficients dB/(cm·MHz) for acoustic waves from different probes in water and in human tissues. According to the attenuation coefficient in water and human body, the corresponding gain compensation is made according to the attenuation.

Specifically, the attenuation coefficient in water is α1= 0.1dB /(cm·MHz); the attenuation in human soft tissue is α2=0.7dB/(cm·MHz), and the attenuation coefficient can be set flexibly as an adjustable and preset parameter of the system according to the examination site and the doctor's habit.

The compensation gain will be set according to the attenuation coefficient. However, t there is usually a water coupling between the probe acoustic window and the tissue part, and the spacing is not fixed. That is, the attenuation in the depth direction is segmented, the first segment is the attenuation α1 in water and the second segment is the attenuation α2 in a human body. In order to design a TGC curve, it is necessary to know the range of depth positions in water and in tissue.

The initial TGC curve is usually designed as an attenuation curve in water or an attenuation curve in tissue. Updating the TGC curves described in the subsequent embodiments of this application, are all processed on the initial TGC curve.

Secondly, locating a depth region containing a tissue image according to the current frame image, and calculating a depth range of a tissue part in the current frame image, to obtain a depth range of a far field segment containing the tissue part and a depth range of a water coupling segment in a near field segment by means of signal and image processing. Due to a larger difference between a gray scale of the image in water and in tissue, a tissue area and a background (low echo area) are distinguished by gray scale or texture during image processing so as to obtain the depth range of the tissue area. Out of this depth range, and before this depth range (near the near-field segment) it can be considered to be a low attenuation coefficient α1 area in water, and in this depth range it is a high attenuation coefficient α2. Corresponding to the depth direction, the appropriate and smooth gain compensation is made.

When the size of the detected tissue depth area is smaller than a preset value, no update is performed. When the detected tissue depth area is too small, it may be a floating object in water and cannot be used as an observation object, and it may be a narrow surface and its depth range is shallower, and its range may not reach the segment interval of the TGC curve, thus losing the meaning of compensation.

Lastly, according to the depth range segment of the water coupling segment and the depth range of the tissue part segment, and an attenuation difference between in water and in a human body, a corresponding full-depth attenuation curve is obtained, and a corresponding TGC curve is updated. The TGC curve obtained from the previous frame processing is applied in next frame processing.

When the tissue part is unchanged and the probe acoustic window is far away from or near the tissue part, the depth segment of the water coupling interval and tissue area is obtained in real time to update the TGC curve in real time.

Further in some embodiments of this application, different attenuation coefficients dB/(cm MHz) can be preset according to the attenuation of ultrasound by different probes and different tissue parts.

Further in some embodiments of this application, a blind area of the probe is also distinguished when the tissue area and the background area are distinguished. The blind area of the probe can be distinguished according to a position in the image corresponding to a physical size of the blind area corresponding to a probe model obtained by predetermined probe identification. In the embodiment of this application, according to a size of the blind area corresponding to the obtained probe model, setting a near field area as a gain attenuation area (the gain coefficient of TGC < 1) for subsequent reduction of a highlighted blind area signal, and setting a far field as a gain compensation area (the gain coefficient of TGC > 1) for amplifying a weak tissue signal.

Further in some embodiments of this application, when obtaining the depth range of the tissue part, use a union of depth range value sets of continuous multi-frame tissue as a depth position area of the tissue and furthermore designing a stable TGC curve for a continuous multi-frame to ensure a gradual change in brightness of inter-frame images and intra-frame images.

Further in some embodiments of this application, in order to ensure real-time adjustment, the system further comprises a computing platform such as FPGA or GPU, which is configured to accelerate image processing and TGC curve update.

Further in some embodiments of this application, the method to adjust and update the TGC curve can be an analog way for adjusting, that is, using a signal amplifier with variable gain to adjust an amplitude of an analog signal before sampling, and can be a digital way for adjusting after acquisition, that is, using the gain coefficient of the curve to adjust a numerical value of data after acquisition.

The following is illustrated in combination with specific embodiments.

Applying a current TGC curve to obtain a current frame image. Obtaining a frequency of a present probe and for a current part to be examined, obtaining a attenuation coefficient (α1) dB /(cm·MHz) of a present probe acoustic wave in water and a attenuation coefficient (α2) dB I(cm MHz) of a present probe acoustic wave in the current part of a human body. Wherein, α1 is the coefficient unrelated to the examined part, and α2 is the coefficient related to the examined part. An initial attenuation coefficient curve is determined according to α1, such as the attenuation curve of acoustic wave in water in Fig. 2. According to the attenuation curve, a TGC curve is designed as the initial gain compensation curve.

Load an image frame and obtain the depth range of the tissue in the frame by means of signal or image processing.

Loading a frame image, and obtaining a depth range of a tissue part in the frame image by means of signal and image processing.

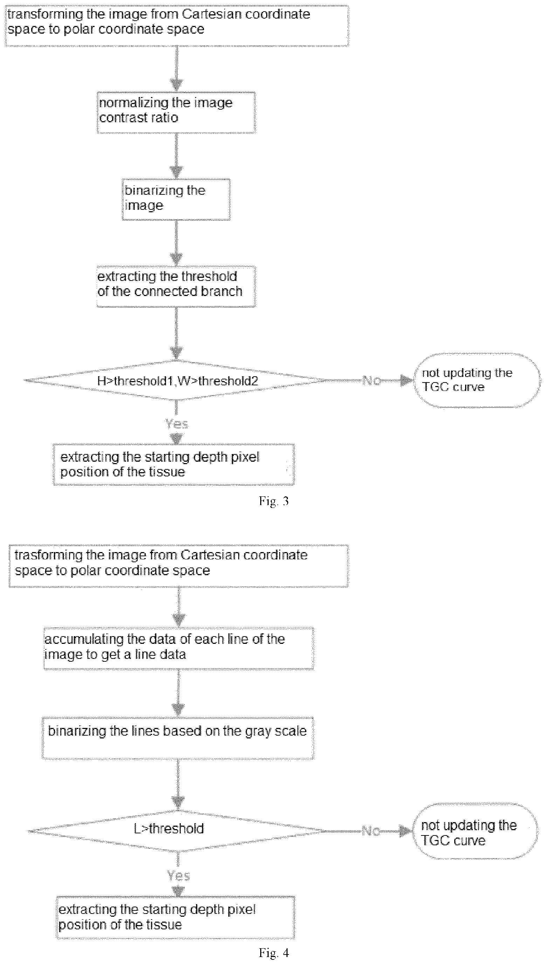

In an embodiment of this application, such as shown in Fig. 3. The image is transformed from Cartesian coordinate space to polar coordinate space when collecting data. Setting the location of the probe to 0 (the physical distance of the probe is known, which can be converted to the pixel position area occupied by the probe on the image). The transformed image is smoothing filtered, and the contrast ratio is normalized line by line. Making dark areas darker and bright areas brighter, that is, the contrast ratio is pulled up by mapping the minimum and maximum values (minGray, maxGray) of the first-line data to (0, 255). Then the image is binarized to distinguish the tissue area and the background area. In particular, this application uses OTSU method for binarization processing. After binarization, multiple connected regions may be obtained to process, and the TGC curve can be updated only when the threshold of each region is determined to ensure that at least one region is satisfied. Threshold 1 is the height threshold of the connected region, that is, in the depth direction, threshold 2 is a supplementary threshold, which is a limitation in angle. The connected regions that satisfies the threshold are grouped together to form a region occupied by the tissue, and then a starting location information of the tissue area in the depth direction is extracted.

Another an embodiment of this application is shown in Fig. 4, the image is transformed from Cartesian coordinate space to polar coordinate space when collecting data. Then the data of each line of the image is accumulated to get a line data. Then the line data is binarized. There are many binarization methods here, and the OTSU method is still used for processing in this embodiment. In this way, we can obtain the depth segment of the tissue area with larger gray scale and the depth segment of the background area with smaller gray scale. Only when the depth segment of the tissue area is greater than a certain length can it be determined as an effective tissue depth, otherwise the TGC curve will not be updated. A starting position of a reserved effective depth segment is a starting depth pixel position of the tissue area in the image.

The former embodiment has the advantages of accurate location of the depth position, while the latter embodiment has the advantages of simple calculation and fast processing speed.

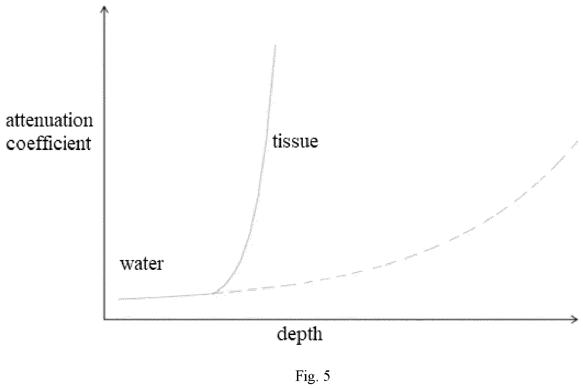

Finally, after the depth range of the tissue is determined, the ultrasonic attenuation curve corresponding to the current frame image is constructed, as shown in Fig. 5. Assuming that the depth of tissue position obtained is 1 to 2cm, as shown in Fig. 5, the position 0 to 1cm is the attenuation curve of water, and the position 1 to 2cm is the attenuation curve of tissue, which forms a composite attenuation curve. In this embodiment, the composite attenuation curve is firstly smoothed curve fitting. Preventing abrupt changes in image brightness due to TGC discontinuity caused by mutations. Finally, according to the smoothed attenuation curve, a corresponding TGC curve is designed. In this embodiment, a simple way to design a TGC curve based on the attenuation curve is to directly compensate the gain of the tissue area segment. TGC usually has multiple depth levels for adjustment, and the corresponding compensation gain can be increased according to the depth level corresponding to the tissue area segment, and the TGC curve is generally required to be a smooth curve to ensure the gradual adjustment of image brightness.

This application also discloses an imaging automatic gain compensation system, which comprises:
a current frame obtaining module, configured to apply a current TGC curve to obtain a current frame image;
an attenuation coefficient setting module, configured to set an attenuation coefficient of a probe acoustic wave in water and an attenuation coefficient of a probe acoustic wave in human tissue;
a signal and image processing module, configured to locate a depth region containing a tissue image according to the current frame image, and obtain a depth range containing a tissue part and a depth range of a water coupling segment;
a gain compensation module, configured to make a corresponding gain compensation by using the attenuation coefficient of a probe acoustic wave in human tissue for the depth range containing the tissue part; and make a corresponding gain suppression by using the attenuation coefficient of a probe acoustic wave in water for the depth range of the water coupling segment;
a TGC curve adjusting and updating module, configured to according to the depth range of the water coupling segment and the depth range of the tissue part segment, and an attenuation difference between in water and in a human body, obtain a corresponding full-depth attenuation curve, and update a corresponding TGC curve.
the attenuation coefficient setting module, is configured to set the attenuation coefficient of a probe acoustic wave in water as α 1, and set the attenuation coefficient of a probe acoustic wave in a human body as α 2, the signal and image processing module is configured to distinguish a tissue area and a background (low echo area) by gray scale or texture during image processing. The signal and image processing module is configured to distinguish between tissue area and background while distinguishing the probe blind area. Furthermore, the blind area of the probe is distinguished according to a position in the image corresponding to a physical size of the blind area corresponding to a probe model obtained by predetermined probe identification; and according to a size of the blind area corresponding to the obtained probe model, a near field area is set as a gain attenuation area (the gain coefficient of TGC < 1) for subsequent reduction of a highlighted blind area signal, and a far field is set as a gain compensation area (the gain coefficient of TGC > 1) for amplifying a weak tissue signal.

The signal and image processing module, is configured to when obtaining the depth range of the tissue part, use a union of depth range value sets of continuous multi-frame tissue as a depth position area of the tissue and furthermore design a stable TGC curve for a continuous multi-frame to ensure a gradual change in brightness of inter-frame images and intra-frame images.
when the depth range area of the detected tissue part segment is smaller than a preset value, the TGC curve adjusting and updating module is configured to do not update the TGC curve.
the TGC curve adjusting and updating module is configured to update the TGC curve by using a signal amplifier with variable gain to adjust an amplitude of an analog signal before sampling, or by using the gain coefficient of the curve to adjust a numerical value of data after acquisition.

In order to ensure real-time adjustment, the system further comprises a computing platform such as FPGA or GPU, which is configured to accelerate image processing and TGC curve update.

The embodiment of this application also discloses a computer-readable storage medium, wherein instructions are stored in the computer-readable storage medium, and when the instructions are run on an ultrasonic endoscopic imaging device, the ultrasonic endoscopic imaging device is caused to perform the imaging automatic gain compensation method described in any of the preceding items.

The embodiment of this application also discloses an ultrasonic endoscope, comprising a memory, a processor, and a computer program stored in the memory and executable on the processor, when the processor executes the computer program, the imaging automatic gain compensation method described in any of the above items is implemented.

The above is a further detailed description of this application combined with specific embodiments, and it cannot be considered that the specific implementation of this application is limited to these descriptions. For those skilled person in this art to which this application belongs, several simple deductions or substitutions can be made without departing from the concept of this application, all of which should be regarded as belonging to the protection scope of this application.

## Claims

1. An imaging automatic gain compensation method, **characterized in that**, comprising the following steps:
step S1, applying a current TGC curve to obtain a current frame image; and setting an attenuation coefficient of a probe acoustic wave in water and an attenuation coefficient of a probe acoustic wave in human tissue;
step S2, locating a depth region containing a tissue image according to the current frame image, and obtaining a depth range of a tissue part segment in a far field segment and a depth range of a water coupling segment in a near field segment by means of signal and image processing;
for the depth range of the tissue part, making a corresponding gain compensation by using the attenuation coefficient of a probe acoustic wave in human tissue;
for the depth range of the water coupling segment, making a corresponding gain suppression by using the attenuation coefficient of a probe acoustic wave in water;
step S3, according to the depth range of the water coupling segment and the depth range of the tissue part segment, and an attenuation difference between in water and in a human body, obtaining a corresponding full-depth attenuation curve, and updating a corresponding TGC curve;
step S4, applying the TGC curve of a previous frame in next frame processing, and repeating steps S1 to S3.

2. The imaging automatic gain compensation method according to claim 1, **characterized in that**:
in step S1, setting an attenuation coefficient of a current probe acoustic wave in water as α 1, setting an attenuation coefficient of a current probe acoustic wave in a current part of a human body to be examined as *α* 2, wherein *α* 2> *α* 1.

3. The imaging automatic gain compensation method according to claim 2, **characterized in that**:
in step S2, when performing image processing, distinguishing a tissue area and a background by gray scale or texture, and distinguishing a blind area of the probe according to a position in the image corresponding to a physical size of the blind area corresponding to a probe model obtained by predetermined probe identification;
according to a size of the blind area corresponding to the obtained probe model, setting a near field area as a gain attenuation area for subsequent reduction of a highlighted blind area signal, and setting a far field as a gain compensation area for amplifying a weak tissue signal.

4. The imaging automatic gain compensation method according to claim 3, **characterized in that**:
in step S2, when obtaining the depth range of the tissue part, using a union of depth range value sets of continuous multi-frame tissue as a depth position area of the tissue, and designing a stable TGC curve for the continuous multi-frame to ensure a gradual change in brightness of inter-frame images and intra-frame images.

5. The imaging automatic gain compensation method according to any one of claims 1 to 4, **characterized in that**:
in step S3, when the depth range area of the detected tissue part segment is smaller than a preset value, the TGC curve being not updated;
updating the TGC curve by using a signal amplifier with variable gain to adjust an amplitude of an analog signal before sampling, or by using a gain coefficient of the curve to adjust a numerical value of data after acquisition.

6. An imaging automatic gain compensation system, **characterized in** comprising:
a current frame obtaining module, configured to apply a current TGC curve to obtain a current frame image;
an attenuation coefficient setting module, configured to set an attenuation coefficient of a probe acoustic wave in water and an attenuation coefficient of a probe acoustic wave in human tissue;
a signal and image processing module, configured to locate a depth region containing a tissue image according to the current frame image, and obtain a depth range containing a tissue part and a depth range of a water coupling segment;
a gain compensation module, configured to make a corresponding gain compensation by using the attenuation coefficient of a probe acoustic wave in human tissue for the depth range containing the tissue part; and make a corresponding gain suppression by using the attenuation coefficient of a probe acoustic wave in water for the depth range of the water coupling segment;
a TGC curve adjusting and updating module, configured to according to the depth range of the water coupling segment and the depth range of the tissue part segment, and an attenuation difference between in water and in a human body, obtain a corresponding full-depth attenuation curve, and update a corresponding TGC curve.

7. The imaging automatic gain compensation system according to claim 6, **characterized in that**:
the attenuation coefficient setting module, configured to set the attenuation coefficient of a current probe acoustic wave in water as *α* 1, and set the attenuation coefficient of a current probe acoustic wave in a human body as *α* 2, wherein *α* 2> *α* 1.

8. The imaging automatic gain compensation system according to claim 7, **characterized in that**:
the signal and image processing module, configured to distinguish a tissue area and a background by gray scale or texture and distinguish a blind area of the probe according to a position in the image corresponding to a physical size of the blind area corresponding to a probe model obtained by predetermined probe identification, when performing image processing; according to a size of the blind area corresponding to the obtained probe model, set a near field area as a gain attenuation area for subsequent reduction of a highlighted blind area signal, and set a far field as a gain compensation area for amplifying a weak tissue signal;
the signal and image processing module, configured to use a union of depth range value sets of continuous multi-frame tissue as a depth position area of the tissue, and design a stable TGC curve for a continuous multi-frame to ensure a gradual change in brightness of inter-frame images and intra-frame images;
when the depth range area of the detected tissue part segment is smaller than a preset value, the TGC curve adjusting and updating module configured to do not update the TGC curve;
the TGC curve adjusting and updating module configured to update the TGC curve by using a signal amplifier with variable gain to adjust an amplitude of an analog signal before sampling, or by using the gain coefficient of the curve to adjust a numerical value of data after acquisition.

9. A computer-readable storage medium, **characterized in that** instructions are stored in the computer-readable storage medium, and when the instructions are run on an ultrasonic endoscopic imaging device, the ultrasonic endoscopic imaging device is caused to perform the imaging automatic gain compensation method according to claims 1-5.

10. An ultrasonic endoscope, **characterized in** comprising a memory, a processor, and a computer program stored in the memory and executable on the processor, when the processor executes the computer program, the imaging automatic gain compensation method according to claims 1-5 is implemented.
